# EUROPEAN PATENT APPLICATION

(11) **EP 4 804 380 A1**
(43) Date of publication of application: **09.09.2026**
(21) Application number: 26163049.5
(22) Date of filing: 06.03.2026
(51) Int. Cl.: H02J 50/10, H02M 7/48

(54) **MULTI-RESONANT ELECTROSURGICAL GENERATOR**

(30) Priority: 06.03.2025 US 202519072782
(71) Applicant: Cilag GmbH International, 6300 Zug (CH)
(72) Inventor: LAFAY, Eric, Cincinnati, 45242 (US)
(74) Representative: Carpmaels & Ransford LLP

(57) **Abstract**

A multi-resonant therapeutic converter includes N+1 branches coupled to a voltage source and a ground, each branch including two transistors. The two transistors of each branch include a high-side transistor coupled to the voltage source and a low side transistor coupled to the high side transistor and the ground. The multi-resonant therapeutic converter includes N resonant tanks each coupled to a respective branch of the N+1 branches and a coil. The coil is further coupled to one of the N+1 branches. The first resonant tank is associated with a first operation and the second resonant tank is associated with a second operation. The coil is selectively powered with the first and second resonant tanks based on states of the transistors of the of the N+1 branches.

## Description

### FIELD OF THE DISCLOSURE

The present disclosure relates generally to power generation during surgical procedures and, more particularly, to an electrosurgical generator for powering surgical instruments and that includes multiple resonant tanks.

### BACKGROUND OF THE DISCLOSURE

Electrical generators are used to power surgical instruments during surgical procedures. These generators are designed to work with particular instruments and meet medical standards to ensure safety, reliability, and precision. For example, monopolar generators may be used with monopolar surgical instruments for cutting and coagulating tissues, requiring a grounding pad to complete an electrical circuit. Bipolar generators, on the other hand, do not require a grounding pad as electrical current can pass through the surgical instrument and reduce the risk of electrical burns. These generators allow precise control over power output to surgical instruments, which can be adjusted based on the type of tissue and desired effect.

Electrical generators are designed to operate at specific frequencies to cut, coagulate, desiccate, or fulgurate tissue. Malfunctioning generators or improper frequency may cause excessive heat generation, which could lead to patient burns. Accordingly, electrosurgical generators should be calibrated to operate at particular frequencies to prevent situations that may compromise safety and effectiveness of a device that receives power from the electrosurgical generator.

### BRIEF DESCRIPTION OF THE DRAWINGS

FIG. 1 is a block diagram of an example surgical system that includes a surgical instrument and a conversion unit that includes a multi-resonant therapeutic converter.
FIG. 2 is multi-resonant circuit of the multi-resonant therapeutic converter of FIG. 1, in accordance with at least one aspect of the present disclosure.
FIG. 3A is a clutching circuit of the multi-resonant therapeutic converter of FIG. 1, in accordance with at least one aspect of the present disclosure.
FIG. 3B is a composite waveform produced by the clutching circuit of FIG. 3A, in accordance with at least one aspect of the present disclosure.
FIG. 4 is an N-channel circuit of the multi-resonant therapeutic converter of FIG. 1, in accordance with at least one aspect of the present disclosure.
FIG. 5 is a schematic flowchart of an example method for controlling power delivered to the surgical instrument of FIG. 1 using the multi-resonant therapeutic converter of FIG. 1.

### DETAILED DESCRIPTION

Embodiments of the present disclosure will now be described in detail with reference to the accompanying Figures. Like elements in the various figures may be denoted by like reference numerals for consistency. Further, in the following detailed description of embodiments of the present disclosure, numerous specific details are set forth in order to provide a more thorough understanding of the claimed subject matter. However, it will be apparent to one of ordinary skill in the art that the embodiments disclosed herein may be practiced without these specific details. In other instances, well-known features have not been described in detail to avoid unnecessarily complicating the description. Additionally, it will be apparent to one of ordinary skill in the art that the scale of the elements presented in the accompanying Figures may vary without departing from the scope of the present disclosure.

Embodiments in accordance with the present disclosure generally relate to power generation during surgical procedures and, more particularly, to an electrosurgical generator for powering surgical instruments and including multiple resonant tanks. As described herein, a resonant tank can be a circuit including reactive components, such an inductor and a capacitor. A resonant tank can operate as a band-pass filter that allows signal within a certain frequency range to pass through the resonant tank, while attenuating signals outside the range. To operate as a band-pass filter, the resonant tank can include reactive components that are arranged in series. Moreover, a series resonant circuit exhibits low impedance at the resonant frequency, such that voltage across the resonant tank experiences low voltage drop relative to the impedance. Conversely, if the resonant circuit does not operate at resonant frequency of the received signal, the resonant tank has a relatively higher impedance. Thus, the higher impedance results in a higher voltage drop across the resonant tank, relative to how far the frequency is from resonance of the resonant tank.

In electrosurgical applications, changes in voltage and frequency can impact the safety and effectiveness of surgical procedures applied by a coupled surgical instrument acting as a load of the electrosurgical circuit. For example, opening or closing the surgical instrument can affect the frequency response of the electrosurgical circuit, such that components, like a resonant tank, receive a different frequency. Accordingly, the frequency of the circuit can deviate from the resonant frequency of the resonant tank such that the impedance of the resonant tank changes, thereby causing a voltage drop across the resonant tank. Because the surgical instrument is configured to operate at a specific voltage, the voltage drop caused by varying frequency can deteriorate the safety and effectiveness of the associated surgical operation. Accordingly, the electrosurgical circuit can be operable to switch between two or more resonant tanks to limit the voltage drop caused by varying frequency.

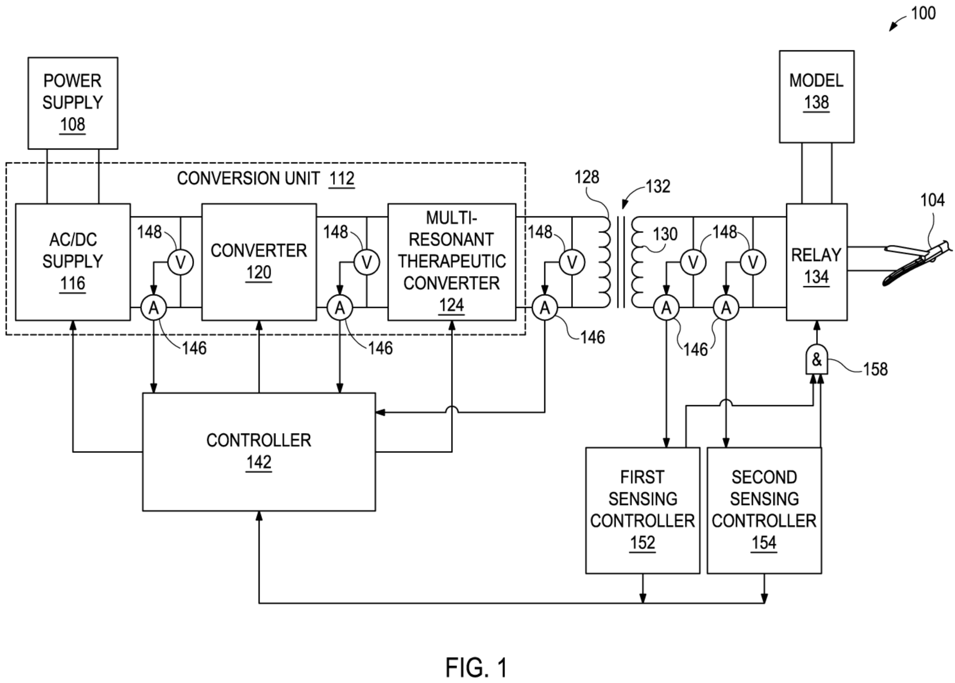

FIG. 1 illustrates a schematic diagram of an example surgical system 100 that may include a surgical instrument 104 operable to perform surgical operations. The surgical instrument 104 may be an electrosurgical unit employed to perform operations of at least cutting and cauterizing tissue. The surgical instrument 104 can perform various operations, such as a therapeutic delivery operation and a pediatric delivery operation. Therapeutic delivery operations can be performed to cut or otherwise operate on adult human tissue, whereas pediatric delivery can be performed to operate on tissue of children or otherwise delicate tissue. To perform these different operations, different power levels may be required by the surgical instrument 104. For example, the therapeutic delivery operation may require power provided between 100 Watts (W) and 400 W, whereas the pediatric delivery operation may require power provided at or below 50 W.

The surgical system 100 may further include a power supply 108 that provides power to the surgical instrument 104. The power supply 108 can be a mains power supply, such as utility or grid power. The voltage provided by the power supply 108 can range from 90 Volts alternating current (Vac) to 240 Vac, which is common in small businesses and residential buildings in North America. Alternatively, the power supply 108 may provide 208/120 Vac or 480/277 Vac.

Regardless of phase, the Alternating Current (AC) power provided may be conditioned to safely and effectively operate the surgical instrument 104 coupled to the power supply 108. Accordingly, the surgical system 100 may include a conversion unit 112 that can receive power from the power supply 108 and provide output power employable by the surgical instrument 104. The conversion unit 112 may include various devices, components, or sub-circuits to convert power provided by the power supply 108. For instance, the conversion unit 112 may include an alternating current/direct current (AC/DC) supply 116 which can convert AC to DC via rectification, filtering, and regulation. Thus, the AC/DC supply 116 can convert AC power to DC power. The DC power generated by the AC/DC supply 116 can be provided to a converter 120, which can be a DC-DC converter capable of stepping up or stepping down the DC voltage provided by the AC/DC supply 116. The voltage converted by the converter 120 can be provided to a multi-resonant therapeutic converter 124 that includes a set of resonant tanks, described in more detail herein below, and may be employed to generate a waveform (e.g., AC) having desired characteristics, such as a specific frequency. To convert voltage, the multi-resonant therapeutic converter 124 can include additional components, discussed further herein, such as switching elements.

The multi-resonant therapeutic converter 124 may provide the waveform to a first coil 128 that may be inductively coupled to a second coil 130 of an isolation transformer 132. As shown in FIG. 1, the first coil 128 can be an output of the conversion unit 112 that provides power to the surgical instrument 104 via the isolation transformer 132. That is, the conversion unit 112 can receive voltage from the power supply 108 and provide voltage to the surgical instrument 104 via the first coil 128. The second coil 130 can be inductively coupled with the first coil 128 such that the second coil 130 can receive power from the conversion unit 112. The first and second coils 128,130 may isolate the surgical instrument 104 from the conversion unit 112 such that sensitive equipment, including the surgical instrument 104, may be protected from electrical surges.

The surgical instrument 104 may be coupled to the second coil 130 via a relay 134. The relay 134 can be a dual pole double throw (DPDT) relay that can switch between two different loads. For example, the surgical instrument 104 may be a first load and a model 138 coupled to the relay 134 may be a second load. The model 138 can be a circuit configured to have an impedance that matches, or at least substantially matches, the impedance of a tissue during a surgical operation. The relay 134 may switch between inputs that includes multiple conversion units 112.

The surgical system 100 may further include a controller 142 that can be employed to control devices of the conversion unit 112 to control power delivery to the surgical instrument 104. The controller 142 can be a microprocessor, or a microprocessor paired with a field programmable gate array (FPGA) to provide control logic to the conversion unit 112. The AC/DC supply 116, converter 120, and multi-therapeutic resonant converter 124 can each include switches that control how voltage is converted at these respective devices. The controller 142 may provide a control signal (e.g., a pulse width modulated signal) that can open and close the switches of the respective devices of the conversion unit 112, thereby controlling how voltage is being converted by the conversion unit 122.

The surgical system 100 may include ammeters 146 and voltmeters 148 at various positions to within the surgical system 100 to measure current and voltage, such as the positions shown in FIG. 1. The current and voltage measured by these ammeters 146 and voltmeters 148 can be provided as feedback to the controller 142, which may control operations of one or more devices within the surgical system 100 based on the received current and voltage measurements. Voltage and current may have desired or expected levels that corresponds to a desired waveform to be provided to the surgical instrument 104. Accordingly, the controller 142 may adjust operation of the conversion unit 112 to adjust the respective voltages and currents to reach the desired/expected levels.

The controller 142 may adjust operations of the AC/DC supply 116, the converter 120, or the multi-resonant therapeutic converter 124, or combinations thereof based on a measured voltage failing to meet a desired voltage. As one example, based on the voltage measured between the multi-resonant therapeutic converter 124 and the converter 120 not meeting the desired voltage, the controller 142 may adjust operations of the AC/DC supply 116 and/or the converter 120 to adjust the voltage measured to the desired voltage. In another example, the voltage measured between the multi-resonant therapeutic converter 124 and the converter 120 may meet the desired voltage at this position, but the voltage measured between the multi-resonant therapeutic converter 124 and the first coil 128 may not be the desired voltage at this position within the surgical system 100. Accordingly, the controller 142 can adjust the multi-resonant therapeutic converter 124 to adjust the voltage (e.g., waveform, frequency, voltage, current, etc.) between the multi-resonant therapeutic converter 124 and the first coil 124. Therefore, the controller 142 can control the waveform provided to the surgical instrument 104 and maintain desired characteristics of the waveform.

The controller 142 may receive feedback signals from a first sensing controller 152 and a second sensing controller 154. Specifically, the sensing controllers 152,154 can receive distinct voltage and current measurements from ammeters 146 and voltmeters 148 positioned between the second coil 130 and the surgical instrument 104. The measurements received by the sensing controllers 152,154 can be provided to the controller 142 as feedback.

Moreover, the sensing controllers 152,154 may each provide a control signal to the relay 134 coupled to the surgical device 104. The first and second sensing controllers 152,154 may be redundant. As such, the control signals by both sensing controllers 152,154 may be provided to a logic device 158 combining the redundant signals to ensure appropriate operation of the relay 134 and surgical device 104. The control signals of the sensing controllers 152,154 may be controlled by the controller 142.

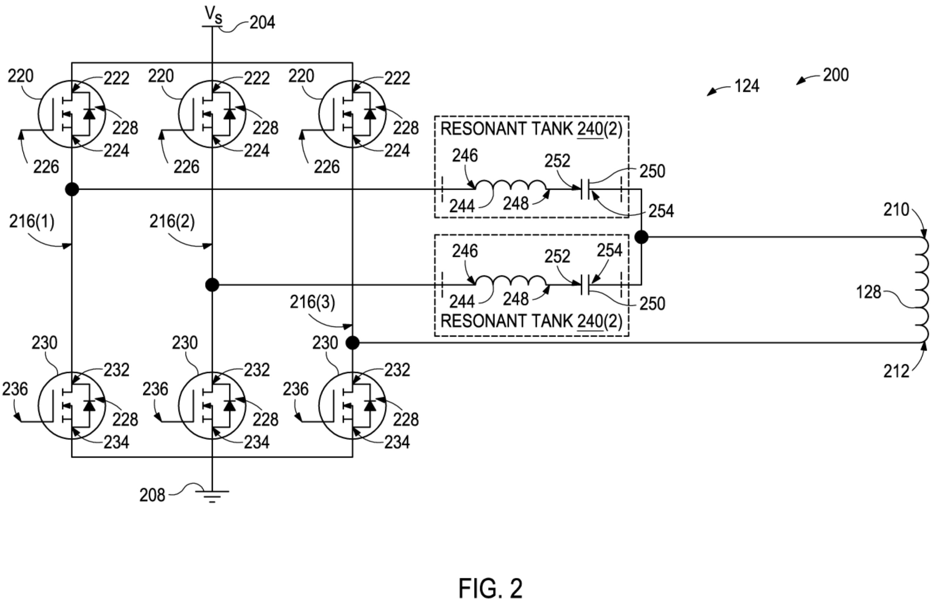

FIG. 2 illustrates a multi-resonant circuit 200 of the multi-resonant therapeutic converter 124 of FIG. 1, in accordance with at least one aspect of the present disclosure. For purposes of simplification of illustration and explanation, the multi-resonant circuit 200 of the multi-resonant therapeutic converter 124 can include the first coil 128 of the surgical system 100 of FIG. 1. However, other embodiments are envisioned in which the multi-resonant circuit 200 of the multi-resonant therapeutic converter 124 does not include the first coil 128. The multi-resonant circuit 200 of the multi-resonant therapeutic converter 124 can receive a source voltage (Vs) 204 from a corresponding converter 120 of the conversion unit 112. In addition, the multi-resonant circuit 200 of the multi-resonant therapeutic converter 124 may be coupled to or otherwise include a ground 208. The first coil 128 can include a positive terminal 210 and a negative terminal 212.

The multi-resonant circuit 200 further includes a plurality of parallel branches 216 between the Vs 204 and the ground 208. Each parallel branch 216 can include a pair of transistors. Each of the transistors may be a Field Effect Transistor (FET), or more specifically, a Metal-Oxide-Semiconductor FET (MOSFET). Each of the transistors can have a drain, a source, and a gate. Each of the transistors are depicted as having a body diode, the body diode being a structure formed between the drain and source.

As shown in FIG. 2, each parallel branch 216 can have a high-side transistor 220 positioned adjacent to the Vs 204, with a given high-side transistor 220 having a drain 222, a source 224 and a gate 226. The drain 222 may be coupled to the Vs 204 and a source 224, while the gate 226 may be coupled to the controller 142 (FIG. 1). The drain 222 may be coupled to the source 224 via a body diode 228. Similarly, each parallel branch 216 can have a low-side transistor 230 having a drain 232, source 234, gate 236, and body diode 238. The drain 232 of the low-side transistor 230 may be coupled to the source 224 of the high-side transistor 220, while the source 234 of the low-side transistor 230 may be coupled to the ground 208. The high-side transistor 220 and low-side transistor 230 can form, or otherwise be a part of, a given parallel branch 216.

As referenced above, the multi-resonant circuit 200 can have multiple parallel branches 216. Specifically, the multi-resonant circuit 200 provided in FIG. 2 includes three parallel branches 216, each denoted using an integer. For purposes of simplification of explanation, the parallel branch 216 located furthest from the first coil 128 can be referred to as the first parallel branch 216(1), the parallel branch 216 closest to the first coil 128 can be referred to as the third parallel branch 216(3), and the parallel branch 216 between the first and third parallel branches 216(1), 216 (3) can be referred to as the second parallel branch 216(2). Similarly, components along the respective parallel branch 216 can be referred to with numerals of the corresponding parallel branch 216. For example, the high-side transistor 220 of the first parallel branch 216(1) can be referred to as the first high-side transistor 220(1).

The transistors (e.g., the high-side transistors 220 and low-side transistors 230) of the parallel branches can be selectively activated by a controller (e.g., the controller 142 of FIG. 1) to provide an alternating current (AC) voltage to a load (e.g., the first coil 128). Therefore, the voltage provided to the load by the multi-resonant circuit 200 may be sinusoidal having a frequency and amplitude. The frequency can be controlled by a switching frequency applied to the transistors by the controller and the amplitude can be controlled by a duty cycle applied to the transistors by the controller. Accordingly, the voltage provided to the load by the multi-resonant circuit 200 may have alternating polarity. Thus, a surgical instrument 104 (FIG. 1) may be bipolar and employ the AC voltage provided by the multi-resonant therapeutic converter 124 to perform surgical operations. In other examples, the voltage provided by the multi-resonant circuit 200 may be a square wave or modified sine wave, each having a frequency and amplitude for a given surgical instrument, like surgical instrument 104 (FIG. 1).

Furthermore, the multi-resonant circuit 200 can have multiple resonant tanks 240. A given resonant tank 240 can include reactive components such that the given resonant tank 240 has a resonant frequency. These reactive components may be arranged to select or filter specific frequencies supplied by the multi-resonant circuit 200. Moreover, a given resonant tank 240 can have an inductor 244 having a first terminal 246 and second terminal 248. The first terminal 246 of the inductor 244 can be coupled to a parallel branch 216 between the high-side transistor 220 thereof and the low-side transistor 230 thereof. More specifically, the first terminal 246 of the inductor 244 can be coupled to the parallel branch 216 between the drain 232 of the low-side transistor 230 and the source 224 of the high-side transistor 220.

Furthermore, the given resonant tank 240 can include a capacitor 250 coupled to the inductor 240 in series. Specifically, the capacitor 250 can have a first terminal 252 that may be coupled to the second terminal 248 of the inductor 240 and a second terminal 254 that may be coupled to the positive terminal 210 of the first coil 128. Accordingly, a resonant tank 240 can be coupled between a parallel branch 216 and the first coil 128. Moreover, the resonant frequency of a given resonant tank 240 can be calculated according to the following expression (1): ${f}_{r} = \frac{1}{2 π \sqrt{L ∗ C}}$ wherein "fᵣ" is the resonant frequency of a given resonant tank 240, "L" is the inductance of the inductor 244, and "C" is the capacitance of the capacitor 250. Thus, the resonant frequency of the given resonant tank 240 may be selected based on inductance and capacitance of the respective inductor 240 and capacitor 250.

To ensure efficient and stable power conversion, the resonant frequency of the resonant tank 240 should match the switching frequency applied to the transistors of multi-resonant therapeutic converter 124. For example, if the transistors are switched at a frequency of 60 Hertz (Hz), the resonant tank 240 should be tuned to have a resonant frequency of 60 Hz. Accordingly, the impedance of the resonant tank 240 may be curtailed as the resonant frequency of the resonant tank 240 aligns more closely with the switching the frequency of the multi-resonant therapeutic converter 124.

Resonant tanks 240 may be referred to with a numeral corresponding to the respective parallel branch 216. For example, a first resonant tank 240(1) may be coupled to the first parallel branch 216(1). Similarly, a second resonant tank 240(2) may be coupled to the second parallel branch 216(2). The third parallel branch 216(3) may be coupled to the negative terminal 212 of the first coil 128. More specifically, the negative terminal 212 of the first coil 128 can couple to the parallel branch 216(3) between the drain 232(3) of the third low-side transistor 230(3) and the source 224(3) of the third high-side transistor 220(3).

The first resonant tank 240(1) may have a resonant frequency of 60 Hz to match a switching frequency of 60 Hz provided to the multi-resonant therapeutic converter 124. However, the second resonant tank 240(2) may have a resonant frequency of 50 Hz, such that the second resonant tank 240(2) has a different (higher) impedance while the multi-resonant therapeutic converter 124 is operating at a switching frequency of 60 Hz. Consequently, the higher impedance of the second resonant tank 240(2) may reduce the output voltage provided by the multi-resonant therapeutic converter 124 compared to the first resonant tank 240(1) due to having a relatively higher impedance. High power or therapeutic delivery may require voltage at 300-480kHz and pediatric delivery at 400-800kHz, such that the resonant tanks 240 can be tuned and selected for a desired operation.

Based on the arrangement of the resonant tanks 240 and parallel branches 216, the controller 142 (FIG. 1) can select a resonant tank 240 for providing voltage (e.g., Vs) to the first coil 128. For instance, the controller 142 may employ the first and third parallel branches 216(1),(3) to select the first resonant tank 240(1). As one example of employing the first and third parallel branches 216(1),(3) to select the first resonant tank 240(1), the controller 142 may turn on (activate) the high-side transistor 220(1) of the first parallel branch 216(1) and the low-side transistor 230(3) of the third parallel branch 216(3), The rest of the transistors may be turned off (de-activated). Activation and de-activation of each of these respective transistors may be referred to collectively as a first state. In this first state, current can flow from Vs 204, through the high-side transistor 220(1), the first resonant tank 240(1), the first coil 128, and the lower-side transistor 230(3), to the ground 208 during a half-cycle of a switching frequency of the multi-resonant therapeutic circuit 124, as will be described in more detail below.

As another example of employing the first and third parallel branches 216(1),(3) to select the first resonant tank 240(1), the controller 142 may turn on (activate) the high-side transistor 220(3) of the third parallel branch 216(3) and the low-side transistor 230(1) of the first parallel branch 216(3) The rest of the transistors may be turned off (de-activated). Activation and de-activation of each of the transistors in this example may be referred to as a second state. Accordingly, current may flow from Vs 204, through the high-side transistor 220(3), the first resonant tank 240(1), the first coil 128, and the low-side transistor 230(1), to the ground 208 during a half-cycle of the switching frequency, as will be described in more detail below. The polarity of the voltage across the first coil 128 (e.g., output voltage) during the first state may be inverted compared to polarity during the second state. For purposes of simplification of explanation, the transient states of these transistors between the first state and a second state for selecting the first resonant tank 240(1) can also be referred to collectively as a state.

As illustrated in FIG. 2, the multi-resonant circuit 200 is constructed to toggle between resonant tanks 240 arranged in parallel. Accordingly, each resonant tank 240 may be tuned for an individual resonant frequency, which may be adjusted by specific selection of inductors 244 and capacitors 250.

Moreover, individual transistors can be controlled with FPGAs and microcontrollers, which may have six or more PWM timer circuits. Therefore, the controller 142 and multi-resonant therapeutic converter 124 may be employed to select between multiple resonant tanks 240 to adjust for various levels of desired output power.

Moreover, PWM can control the output voltage using a duty cycle. A duty cycle is a fraction of one period in which a signal, system, or component is active, such as a transistor. A duty cycle D can represent a fraction or percentage of the period, which may be the switching frequency of the multi-therapeutic resonant converter 124. A duty cycle of one may allow the output voltage to be equal to an input voltage (e.g., Vs 204), whereas a duty cycle less than one (e.g., 0.5) may result in reduced output voltage. A duty cycle between zero and one can introduce harmonic frequencies to the switching frequency (e.g., fundamental frequency). These harmonic frequencies may be filtered by the resonant tanks 240, which allow voltage to pass at the switching frequency.

The first resonant tank 240(1) can have a resonant frequency associated with relatively higher, or "first" power, such as about 100-400W, for example, which may be employable by the controller 142 during therapeutic delivery operations. The second resonant tank 240(2) can have a resonant frequency associated with relatively lower, or "second" power less than the first power, such as about 50 W or less, for example, which may be employable by the controller 142 during pediatric delivery operations. The controller 142 may employ the second and third parallel branches 216(2)-(3) to select the second resonant tank 240(2).

Considering the therapeutic and pediatric delivery operations may operate on different tissues having different impedances that require different levels of power, the switching frequency may be impacted and reduce the effectiveness of a given resonant tank 240. By employing the multi-resonant circuit 200 for the multi-resonant therapeutic converter 124, the multi-resonant therapeutic converter 124 can allow the associated surgical system (e.g. surgical system 100 (FIG. 1)) to perform multiple operations with different resonant tanks 240, ensuring safe and effective operations. For example, at higher or "first" resistance, a lower or "first" switching frequency may reduce switching losses and improve efficiency, whereas at relatively lower or "second" resistance less than the first resistance, a higher or "second" switching frequency greater than the first switching frequency may be beneficial for more dynamic responses and reduced harmonic distortion.

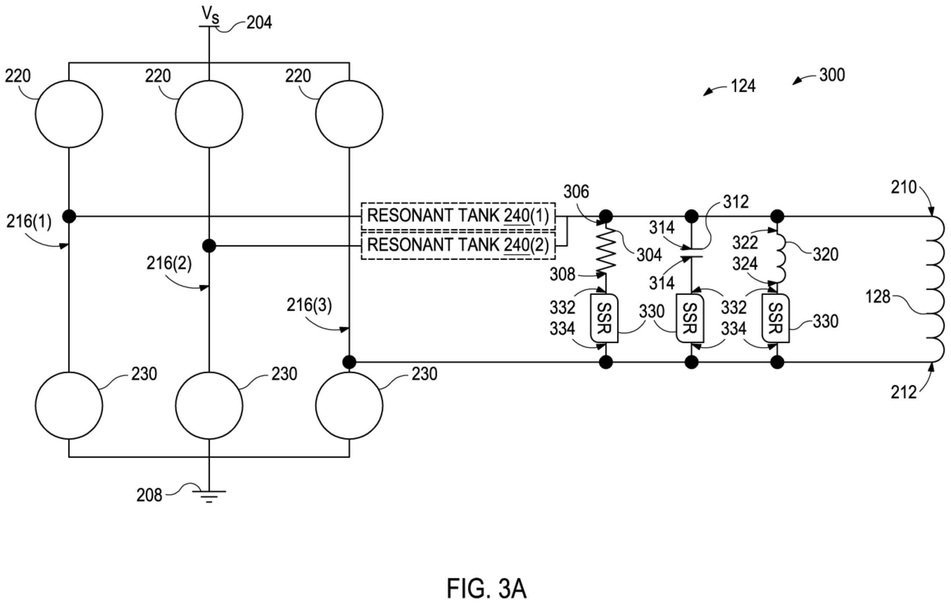

FIG. 3A illustrates a clutching circuit 300 of the multi-resonant therapeutic converter 124, in accordance with at least one aspect of the present disclosure. The clutching circuit 300 may be similar in some respects to the multi-resonant circuit 200 of FIG. 2, and therefore may be best understood with reference thereto, where like numerals will correspond to like components not described again in detail.

The clutching circuit 300 may include a clutching resistor 304 positioned in parallel with the first coil 128. The clutching resistor 304 may have a first terminal 306 coupled to the positive terminal 210 of the first coil 128 and a second terminal 308 coupled to the negative terminal 212 of the first coil 128. The clutching resistor 304 may help stabilize voltage across the first coil 128 by reducing voltage fluctuations across the first coil 128, thereby stabilizing the voltage provided to the surgical instrument 104 (FIG. 1). Further, the clutching resistor 304 may share current with the first coil 128 such that the clutching resistor 304 can reduce overall current through the first coil 128, thereby extending the lifespan of the first coil 128 and surgical instrument 104 (FIG. 1). Furthermore, the clutching resistor 304 may dampen oscillations and reduce effects of resonance in AC circuits such as the clutching circuit 300, which may improve overall stability and performance of the clutching circuit 300.

The clutching circuit 300 can further including reactive clutching components. For instance, as illustrated in FIG. 3A, the clutching circuit 300 may include a clutching capacitor 312 coupled in parallel to the first coil 128. The clutching capacitor 312 can have a first terminal 314 coupled to the positive terminal 208 of the first coil 218 and a second terminal 316 coupled to the negative terminal 210 of the first coil 128. The clutching capacitor 312 may filter out high-frequency noise and smooth voltage fluctuations. The clutching capacitor 312 may also store and release energy to maintain a steady (uniform) voltage across the first coil 128, which may smooth ripple voltage. The clutching capacitor 312 may also reduce overall reactive power in the clutching circuit 300 by compensating for inductive loads, thereby improving efficiency of the clutching circuit 300.

The clutching circuit 300 may include a clutching inductor 320 coupled in parallel to the first coil 128. The clutching inductor 320 can have a first terminal 322 coupled to the positive terminal 208 of the first coil 128 and a second terminal 324 coupled to the negative terminal 210 of the first coil 128. The clutching inductor 320 may reduce low-frequency interference and stabilize current through the first coil 128. The clutching inductor 320 may also be used to select or reject specific frequencies to tune reactance of the first coil 128. The clutching inductor 320 may also temporarily store energy to protect the first coil 128 from sudden changes in voltage. The clutching inductor 320 may also reduce the reactance of the first coil 128 to assist with impedance matching between the first coil 128 and a load (e.g., the surgical instrument 104 (FIG. 1)).

The clutching components of the clutching circuit 300 may be employed to enhance overall performance of the multi-therapeutic resonant converter 124 by providing stabilization, filtering, impedance matching, and protection. These clutching components, in parallel, may optimize power transfer to a load (e.g., surgical instrument 104 (FIG. 1)) by minimizing reflections from the load. Accordingly, impedance can be matched to the load.

The impedance of the load may change in response to the specific surgical instrument 104, as well as operations performed by the surgical instrument 104 represented by the load. Therefore, each of the clutching components may be coupled in series with a solid state relay (SSR) 330, which may be a triac or another similarly controllable electrical components.

As illustrated in FIG. 3A, the second terminal 308 of the clutching resistor 304 may be coupled to a first terminal 332 of a first SSR 330 and a second terminal 334 of the first SSR 330 may be coupled to the negative terminal 210 of the first coil 128. The clutching capacitor 312 and clutching inductor 320 may each be coupled to a respective second and third SSR 300 in a similar manner. The SSRs 330 can be turned "off" and "on" by the controller 142 (FIG. 1) to control the impedance of the clutching components and first coil 128 to match the impedance of the load, which may change during the course of an operation.

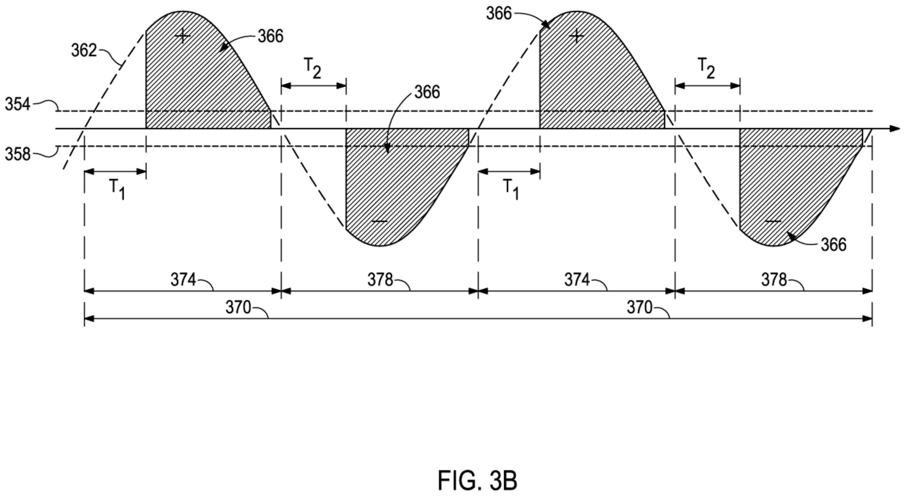

FIG. 3B illustrates a composite waveform 350 that may be measured across the clutching inductor 320 of the clutching circuit 300. The waveform 350 includes a first holding current 354 and a second holding current 358 that are constant, or at least substantially constant. The first holding current 354 is a first polarity (*e.g.* positive) and the second holding current 358 is a second polarity opposite the first polarity (*e.g.* negative). These holding currents 354,358 may allow triacs, such as the SSRs 330 of the clutching circuit 300 (FIG. 3A) to turn on (activate) and off (deactivate) and maintain a conducting state after being turned on. The composite waveform 350 may further include a supply waveform 362 characterizing voltage provided by parallel branches 216 of the multi-resonant therapeutic converter 124. The composite waveform 350 may also include an output voltage 366 measured across the clutching inductor 320. The output voltage 366 and supply waveform 362 may be sinusoidal signals with two full-wave cycles 370, with each full-wave cycle 370 including a first half-wave cycle 374 and a second half-wave cycle 378.

As previously referenced, half wave cycles of the switching frequency may have an associated duty cycle defining an amount time that the respective transistors are active to control voltage. Similarly, an SSR 330 can be activated by the controller 142 (FIG. 1) to perform phase control of the voltage provided to the first coil 128. For instance, referring to FIG. 3A, an SSR 330 may be activated after a first period of time T1 beginning with a start of the first half-wave cycle 374. At the end of the first period of time T1, the SSR 330 may be activated and voltage (e.g., the supply waveform 362) may be provided until falling below the first holding current 354. At the start of the second half-wave cycle 378, a second period of time T2 may elapse. The SSR 330 may not be reactivated until the end of the second period of time T2. At the end of the second period of time T2, the SSR 330 may be activated until the supply waveform 362 falls below the second holding current 358. When the SSR 330 is activated, the inductor 320 and SSR 330 conduct current to decrease magnetizing inductance of the isolation transformer 132 (FIG. 1).

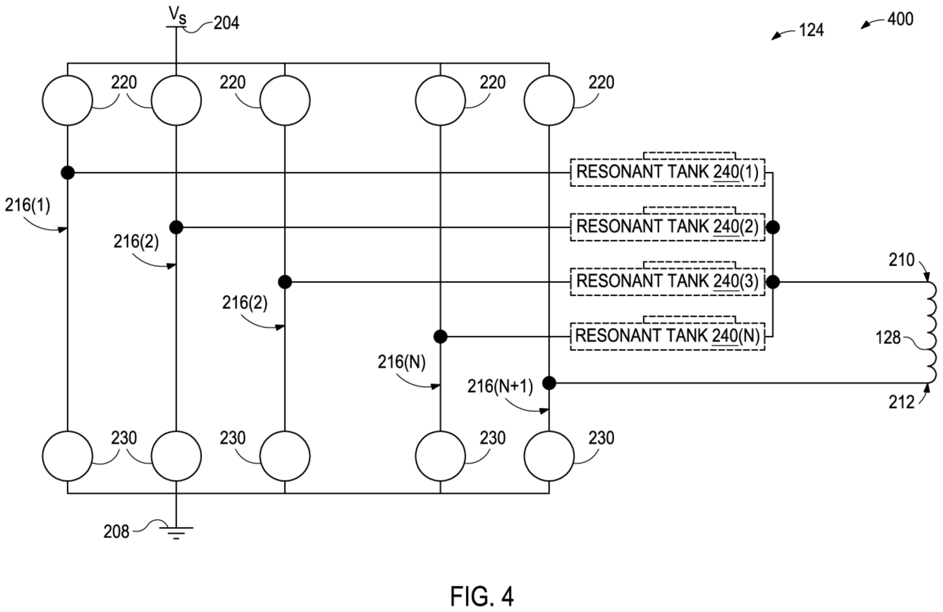

FIG. 4 is an N-channel circuit 400 of the multi-resonant therapeutic converter 124, in accordance with at least one aspect of the present disclosure. The N-channel circuit 400 may be similar in some respects to the multi-resonant circuit 200 of FIG. 2 and the clutching circuit 300 of FIG. 3A, and therefore may be best understood with reference thereto, where like numerals will correspond to like components not described again in detail.

The N-channel circuit 400 can have N+1 parallel channels 216, with N corresponding to the number of resonant tanks 240 within the N-channel circuit 400 while one parallel branch 216 (*e.g.* the N+1 parallel branch) may not have a corresponding resonant tank. N may be any suitable integer, such as two, three, four, five, or six. As an example, an N-channel circuit 400 wherein "N" is equal to two may be the same, or substantially the same, as the multi-resonant circuit 200 provided in FIG. 2.

Similar to the multi-resonant circuit 200 (FIG. 2), the resonant tanks 240 of the N-channel circuit 400 can be coupled to a parallel branch 216. For example, unlike the multi-resonant circuit 200 (FIG. 2), the third resonant tank 240(3), as illustrated in FIG. 4, may be coupled to the third parallel branch 216(3), between the drain 232(3) of the third low-side transistor 230(3) and the source 224(3) of the third high-side transistor 220(3).

In the N-channel circuit 400 and the multi-resonant circuit 200 (FIG. 2), the last parallel branch 216 (e.g., the parallel branch 216 closest to the first coil 128) may be coupled to the negative terminal 210 of the first coil 128. However, other embodiments are envisioned in which any of the parallel branches 216 may be coupled to the negative terminal 210 of the first coil 128 in lieu of the parallel branch 216 closest to the first coil 128, such as the first parallel branch 216(1).

As previously discussed with respect to the multi-resonant circuit 200 of FIG. 2, which may be considered an N-channel circuit where "N" equals two, a first resonant tank 240(1) may be employed during therapeutic delivery operations, whereas a second resonant tank 240(2) may be employed during pediatric delivery operations. That said, "N" may correspond to the number of different operations performable by a surgical instrument (e.g., surgical instrument 104 (FIG. 1)). Accordingly, the N-channel circuit 400 may provide N resonant tanks 240(1)-(N) which may be associated with N number of different operations performable by the surgical instrument 104 (FIG. 1)..

The switching frequency of the circuit 400 may change in response to an operation performed by the surgical instrument 104 (FIG. 1). For instance, the switching frequency of the circuit 400 may need to be changed (adjusted) by the controller 142 based on a change in the load on the circuit 400, such as due to a change in impedance of tissue being operated on by the surgical instrument 104. During a therapeutic delivery operation, the surgical instrument 104 may be employed to cut one or more types of tissue (e.g., muscle, fat, connective tissue), where each type of tissue may have a varying electrical impedance. Moreover, the surface area of the tissue in contact with the surgical instrument 104 may change during the operation, which may also change the impedance of the tissue. Movement of the surgical instrument 104, operational settings (e.g., cut, coagulation, blend, etc.), electrode condition, and patient factors such as body composition and hydrations levels, may also cause changes in impedance. Changes in impedance (e.g., resistance, inductance, capacitance) may require differing switching frequencies of the circuit 400 to ensure that the circuit 400 can handle the varying power demand by the surgical instrument 104. Impedance of the tissue may cause a lag in current, necessitating adjustments to the switching frequency to maintain efficiency and performance.

Although the impedance of the tissue may change during the operation, it may be desirable to maintain, or at least substantially maintain, the desired power to apply to the surgical instrument 104 (FIG. 1) . For example, it may be desirable to deliver 200 W to the surgical instrument during a therapeutic delivery operation. When utilizing the N-channel circuit 400 (FIG. 2), the first resonant tank 240(1) may be employed, which may have a resonant frequency associated with Vs at relatively higher voltages (e.g., 100-400W) associated with therapeutic delivery operations.

During the therapeutic delivery operation, the impedance of the tissue may change, as discussed above. Therefore, the first resonant tank 240(1) may no longer resonate with the switching frequency of the N-channel circuit 400. Accordingly, for the above-described example, the power applied to the surgical instrument 104 may drop below the desired 200 W level due to the change in switching frequency. Changes in frequency may be measured by one or more of the sensing controllers 152,154 (FIG. 1). In some embodiments, the ammeters 146 (FIG. 1) and/or voltmeters 148 (FIG. 1) can be digital multi-meters (DMMs) or devices that can measure multiple electrical parameters, including frequency. The measured frequency may be provided to the controller 142 as a feedback signal. Moreover, the voltage and current may be provided to the controller 142 as a feedback signal, which may be influenced by the frequency of the N-channel circuit 400 and resonant frequency of the resonant tank 240.

Based on a change of impedance of the tissue, for the above-described example, the output power may drop to 100 W, which is below the desired output power (200 W) to provide to the surgical instrument 104 in this example. Based on this change, the controller 142 may employ a different resonant tank 240, such as the third resonant tank 216(3), which may have a resonant frequency closer to the frequency of the N-channel circuit 400 compared to the first resonant tank 216(1), which may thereby cause the power delivered to the surgical instrument 104 to return to the desired power. Thus, the N-channel circuit 400 may be employed to operate a surgical instrument 104 effectively and safely during operations.

The multiple resonant tanks 240 of the N-channel circuit 400 can be selected and actuated simultaneously to provide the desired power to the surgical instrument 140. That is, a multi-resonant therapeutic converter 124 can employ two or more resonant tanks 240 to produce an output signal that is a combination of signals output by the two or more resonant tanks 240. As one example, a first resonant tank 240(1) may resonate at the switching frequency, thereby providing minimal impedance to a current (e.g., a signal) to pass through the first resonant tank 240(1). A second resonant tank 240(2) may be tuned to a different frequency than resonance of the first resonant tank 240(1) and switching frequency, such that the second resonant tank 240(2) may provide impedance to the signal. While the second resonant tank 240(2) may not resonate as strongly as the first resonant tank 240(1), the second resonant tank 240(2) may still influence the overall signal experienced by the first coil 128. For example, the second resonant tank 240(2) can provide distortion or modulation to the output signal experienced by the first coil 128, although the signal provided by the first resonant tank 240(1) will provide greater contribution than the signal provided by the second resonant tank 240(2). However, the second resonant tank 240(2) may provide damping effects to the transient output signal to obtain signals required by specifical surgical instruments 104 and operations. In another example, a combination signal can be produced by two or more multi-resonant therapeutic converters 124, such as the multi-resonant circuit 200 in combination with the N-channel circuit 400. In other examples, the multi-therapeutic converter 124 may be a multi-level inverter that employs two or more resonant tanks 240 to generate a combination signal.

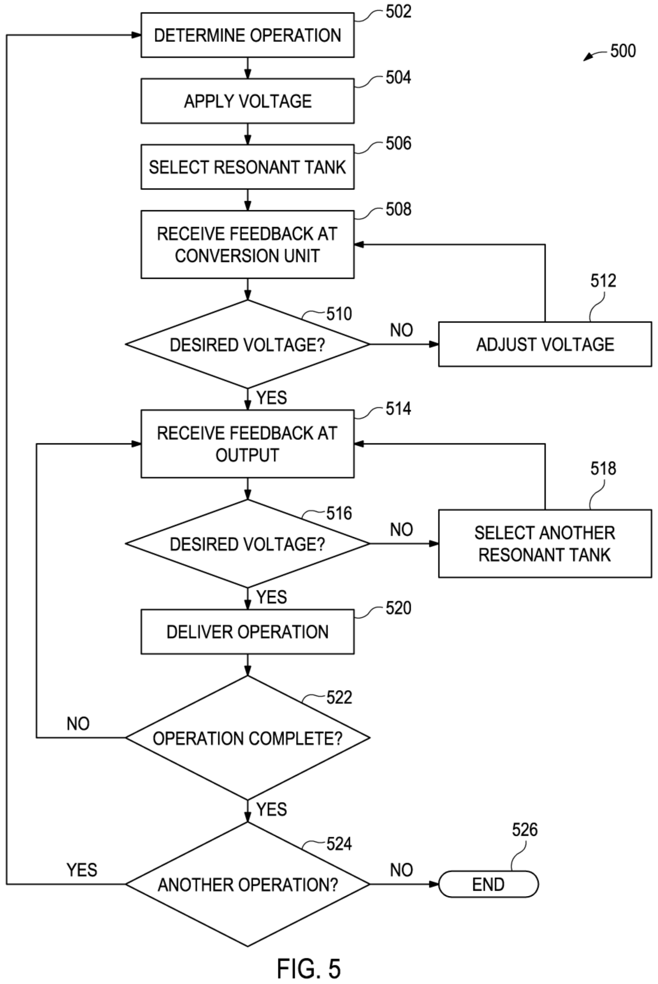

In view of the structural and functional features described above, example methods will be better appreciated with reference to FIGS. 1-4. While, for purposes of simplicity of explanation, the example method of FIG. 5 is shown and described as executing serially, it is to be understood and appreciated that the present examples are not limited by the illustrated order, as some actions could, in other examples, occur in different orders, multiple times and/or concurrently from that shown and described herein. Moreover, it is not necessary that all described actions be performed to implement the methods, and conversely, some actions may be performed that are omitted from the description.

FIG. 5 is a schematic flowchart of an example method 500 for controlling power delivered to a surgical instrument using a multi-resonant therapeutic converter, in accordance with at least one aspect of the present disclosure. The method 500 may be implemented by the controller 142 (FIG. 1). Thus, reference can be made to FIGS. 1-4 in addition to FIG. 5.

The method 500 may include determining an operation to perform with a surgical instrument, as at step 502. For instance, at step 502, the controller 142 may receive an input from a user indicative of a desired operation to perform with the surgical instrument 104, such as a therapeutic delivery operation or pediatric delivery operation.

The method 500 may further include applying a voltage, as at step 504. For instance, at step 504, the controller 142 may allow voltage to be provided to the conversion unit 112 via the power supply 108. More specifically, the controller 142 may operate the devices of the conversion unit 112 (*e.g.* the AC/DC supply 116, converter 120, and multi-therapeutic resonant converter 124) to supply power to the surgical instrument 104, as described elsewhere herein.

The method 500 may further include selecting a resonant tank, as at step 506. For instance, at step 506, the controller 112 may select a resonant tank 240 of the multi-therapeutic converter 124 to provide power to the surgical instrument 104 corresponding to the operation determined at step 502. For example, the controller may select the first resonant tank 240(1) by powering "on" and "off" transistors of the first parallel branch 216(1) associated with the first resonant tank 240(1), as well as transistors associated with the last parallel branch 216(N+1), as described elsewhere herein. The operation selected at step 502 may be associated with a particular resonant tank. For instance, the first resonant tank 240(1) may be associated with the therapeutic operation. Moreover, selecting the resonant tank 240 at step 506 may be performed by the controller 142 contemporaneously with controlling the conversion unit 112 to provide power at step 504.

The method 500 may further include receiving feedback at a conversion unit, as at step 508. For instance, at step 508, the controller 142 may receive feedback from the conversion unit 112. More specifically, the controller 142 may receive feedback (e.g. voltage and/or current measurement) from the ammeters 146 and the voltmeters 148, such as those positioned between at least the multi-resonant therapeutic converter 124 and the converter 120.

The method 500 may further include determining if a measured voltage is a desired voltage, as at step 510. For instance, at step 510, the controller 142 may determine whether the voltage measured at step 508 is at a desired voltage level corresponding to the operation determined at 502. Desired voltage levels for corresponding operations may be stored in a memory of the controller 142 and may be retrieved by the controller 142, such as at, during, or prior to step 510. The controller 142 may compare the measured voltage to the desired voltage to determine if the measured voltage is at, or within an acceptable threshold from, the desired voltage.

Based on the controller 142 determining that desired voltage level is not measured at step 510, the method 500 may proceed along the "NO" branch to step 512, at which the controller 142 may adjust the conversion unit 112 to meet the desired voltage level at 512. Accordingly, steps 508-512 can be repeated until the desired voltage level is met at step 508. It should be noted that the voltage measured at step 508 may characterize the voltage before reaching the multi-therapeutic resonant converter 124. Accordingly, the controller 142 may adjust the AC/DC supply 116 and converter 120 at step 512 at adjust the voltage. This voltage level may correspond to Vs 204 of the multitherapeutic converter 124.

Based on the controller 142 determining that desired voltage level is measured at step 510, the method 500 may proceed along the "Yes" branch to step 514, at which the controller 142 may receive feedback from measuring voltage and current (e.g., power) from ammeters 146 and voltmeters 148 positioned between at least the multi-resonant therapeutic converter 124 and the surgical instrument 104. Moreover, feedback received at step 514 may also be from the one or more of the sensing controllers 152,154.

The method 500 may further include determining if a measured voltage is a desired voltage, as at step 516. For instance, at step 516, the controller 142 can determine whether the measured voltage is at a desired voltage level by comparing the voltage (e.g., power) delivered to the surgical instrument 104 to the desired voltage level.

Based on the controller 142 determining that desired voltage level is not measured at step 516, the method 500 may proceed along the "NO" branch to step 518, at which the controller 142 can select another resonant tank 240. For instance, the surgical instrument 104 may not be operating at a frequency or an impedance that results in the resonant frequency of the first resonant tank 240(1), which may have been selected at step 506. Thus, the controller 142, at step 518, may select another resonant tank 240 that has a resonant frequency that allows delivery of power at the desired level, such as the third resonant tank 240(3).

Based on the controller 142 determining that voltage measured at step 514 is the same, or substantially similar, to the desired voltage at step 516, the method 500 may proceed along the "Yes" branch to step 520, at which the controller 142 may deliver the determined operation, as determined at step 502.

The method 500 may further include determining if the determined operation has been completed, as at step 522. For instance, the controller 104 may determine if the determined operation has been completed by receiving a signal from the surgical instrument characterizing termination of the operation. Alternatively, the controller 142 may determine the determined operation is completed based on received voltage and/or current measurements.

Based on the controller 142 determining that operation has not been completed at step 522, an operator or user can continue to operate the surgical instrument 104. Furthermore, the method 500 may proceed along the "No" branch back to step 514 to measure the voltage provided to the surgical instrument 104 and then determine, at step 516, whether voltage provided to the surgical instrument 104 continues to be at a desired level. For instance, during step 520, the impedance of the tissue may change in response to delivering the operation. Therefore, the frequency of the electrical circuit 400 of the multi-resonant therapeutic converter 124 may be different than the third resonant converter 240(3), which may have been selected at step 518 in the previous iteration of the method 500. The voltage measured at step 514 may also be determined to be different than the desired voltage level at step 516 in the present iteration. Accordingly, the controller 142 may select another available resonant tank 240 at step 518, such as the first resonant tank 240(1) or the fourth resonant tank 240(4), as examples.

Based on the controller 142 determining that operation has been completed at step 522, the controller 142 may proceed along the "Yes" branch to step 524 to determine whether another operation is required. For instance, the controller 142 may receive feedback characterizing user input selecting another operation, similar to what was done at step 502. Based on receiving the user input, the method 500 can return to step 502 and determine the operation to be performed with the multi-resonant therapeutic converter 124 and surgical instrument 104. For example, a user may decide to employ the surgical instrument 104 to cut softer tissue relative to tissue cut during the therapeutic delivery operation. As such, the user may utilize a pediatric delivery or soft tissue operation of the surgical instrument 104, which requires lower voltage compared to the therapeutic delivery operation, as discussed elsewhere herein. Accordingly, the user may provide an input to the controller 142 at steps 524, 502, indicating that a pediatric delivery or soft tissue operation of the surgical instrument 104. Based on the input, the method 500 may be completed again using the same controller 142, multi-therapeutic resonant converter 124, and surgical instrument 104, as described herein above.

Based on receiving an input from the user at step 524, failing to receive an input after a predetermined amount of time at step 524, or determining the power supply 108 has been turned off (de-energized), the method 500 may proceed along the "No" branch to step 526, at which the controller 142 may terminate the method 500.

Accordingly, the foregoing systems may be employed to provide stable and effective voltage to a surgical instrument. The foregoing systems (e.g., circuits) are controllable to select resonant tanks according to demands of the surgical instrument required during various operations. The circuits may have varying frequencies based on switching frequencies and duty cycles employed to provide specific voltages for differing operations, which results in voltage drop in existing systems. This voltage loss is prevented by selecting another resonant tank that resonates at a frequency system corresponding to an operation of the surgical instrument.

Embodiments disclosed herein include:
A. A multi-resonant therapeutic converter, comprising: N+1 branches coupled to a voltage source and a ground, each branch comprising two transistors; and N resonant tanks each coupled to a respective branch of the N+1 branches and a coil, wherein the coil is further coupled to one of the N+1 branches.
B. A surgical system comprising: a surgical instrument operable to perform a first operation and a second operation; a multi-resonant therapeutic converter including a first resonant tank associated with the first operation and a second resonant tank associated with the second operation; and a controller operable to selectively power the surgical instrument with the first and second resonant tanks.
C. A method, comprising: determining, by a controller, an operation to perform with a surgical instrument; applying current, through a first resonant tank, to the surgical instrument based on the determined operation; receiving, by the controller, an input indicative of a voltage applied to the surgical instrument; and applying current, through a second resonant tank, to the surgical instrument based on the received input.

Each of embodiments A through C may have one or more of the following additional elements in any combination: Element 1: wherein the two transistors comprise: a high-side transistor coupled to the voltage source; and a low side transistor coupled to the high side transistor and the ground. Element 2: wherein at least one of the N resonant tanks comprises: an inductor coupled to at least one of a source of the high side transistor the drain of the low side transistor; and a capacitor coupled to the inductor and the coil. Element 3: wherein at least one of the high-side transistor or the low-side transistor comprises a gate operable to receive a control signal. Element 4: wherein the N resonant tanks comprise a first resonant tank and a second resonant tank, wherein the first resonant tank conducts current based on the high-side and low-side transistors being in a first state.

Element 5: wherein the second resonant tank abstains from conducting current based on the high-side and low-side transistors being in the first state. Element 6: wherein, based on the high-side and low-side transistors being in a second state: the second resonant tank conducts current; and the first resonant tank abstains from conducting current. Element 7: wherein the N resonant tanks further comprise a third resonant tank that conducts current based on the high-side and low-side transistors being in a third state. Element 8: wherein the first and second resonant tanks abstain from conducting current based on the high-side and low-side transistors being in the third state. Element 9: further comprising a clutching component coupled to the coil in parallel. Element 10: wherein the clutching component comprises a clutching inductor.

Element 11: further comprising a clutching capacitor coupled in parallel to the coil and the clutching inductor. Element 12: further comprising a clutching resistor coupled in parallel to the coil, the clutching inductor, and the clutching capacitor. Element 13: further comprising a solid-state relay (SSR) coupled in series with the clutching component. Element 14: wherein the controller is operable to receive an input indicative of an applied voltage to the multi-resonant therapeutic converter. Element 15: wherein the controller is operable to receive an input indicative of a voltage applied to the surgical instrument. Element 16: wherein the multi-resonant therapeutic converter further includes a third resonant tank, and wherein the controller is operable to: detect a change in frequency of the voltage applied to surgical instrument while performing the first operation; and power the surgical instrument with the third resonant tank based on the change. Element 17: further comprising: detecting, by the controller, a change in frequency of the voltage applied to surgical instrument while performing the operation; and applying current, through a third resonant tank, based on the change.

In particular, the present invention provides: a method, comprising: determining, by a controller, an operation to perform with a surgical instrument; applying current, through a first resonant tank, to the surgical instrument based on the determined operation; receiving, by the controller, an input indicative of a voltage applied to the surgical instrument; and applying current, through a second resonant tank, to the surgical instrument based on the received input. Optionally this method further comprises detecting, by the controller, a change in frequency of the voltage applied to surgical instrument while performing the operation; and applying current, through a third resonant tank, based on the change.

By way of non-limiting example, exemplary combinations applicable to A through C include: Element 1 with Element 2; Element 2 with Element 3; Element 1 with Element 4; Element 4 with Element 5; Element 4 with Element 6; Element 4 with Element 7; Element 7 with Element 8; Element 3 with Element 9; Element 9 with Element 10; Element 10 with Element 11; Element 11 with Element 12; Element 9 with Element 13; and Element 15 with Element 16.

The terminology used herein is for the purpose of describing particular embodiments only and is not intended to be limiting of the invention. As used herein, for example, the singular forms "a," "an," and "the" are intended to include the plural forms as well, unless the context clearly indicates otherwise. It will be further understood that the terms "contains", "containing", "includes", "including," "comprises", and/or "comprising," and variations thereof, when used in this specification, specify the presence of stated features, integers, steps, operations, elements, and/or components, but do not preclude the presence or addition of one or more other features, integers, steps, operations, elements, components, and/or groups thereof.

Terms of orientation used herein are merely for purposes of convention and referencing and are not to be construed as limiting. However, it is recognized these terms could be used with reference to an operator or user. Accordingly, no limitations are implied or to be inferred. In addition, the use of ordinal numbers (*e.g.,* first, second, third, etc.) is for distinction and not counting. For example, the use of "third" does not imply there must be a corresponding "first" or "second." Also, if used herein, the terms "coupled" or "coupled to" or "connected" or "connected to" or "attached" or "attached to" may indicate establishing either a direct or indirect connection, and is not limited to either unless expressly referenced as such.

While the disclosure has described several exemplary embodiments, it will be understood by those skilled in the art that various changes can be made, and equivalents can be substituted for elements thereof, without departing from the spirit and scope of the invention. In addition, many modifications will be appreciated by those skilled in the art to adapt a particular instrument, situation, or material to embodiments of the disclosure without departing from the essential scope thereof. Therefore, it is intended that the invention not be limited to the particular embodiments disclosed, or to the best mode contemplated for carrying out this invention, but that the invention will include all embodiments falling within the scope of the appended claims. Moreover, reference in the appended claims to an apparatus or system or a component of an apparatus or system being adapted to, arranged to, capable of, configured to, enabled to, operable to, or operative to perform a particular function encompasses that apparatus, system, or component, whether or not it or that particular function is activated, turned on, or unlocked, as long as that apparatus, system, or component is so adapted, arranged, capable, configured, enabled, operable, or operative.

## Claims

1. A multi-resonant therapeutic converter, comprising:
N+1 branches coupled to a voltage source and a ground, each branch comprising two transistors; and
N resonant tanks each coupled to a respective branch of the N+1 branches and a coil, wherein the coil is further coupled to one of the N+1 branches.

2. The multi-resonant therapeutic converter of claim 1, wherein the two transistors comprise:
a high-side transistor coupled to the voltage source; and
a low side transistor coupled to the high side transistor and the ground.

3. The multi-resonant therapeutic converter of claim 2, wherein at least one of the N resonant tanks comprises:
an inductor coupled to at least one of a source of the high side transistor the drain of the low side transistor; and
a capacitor coupled to the inductor and the coil.

4. The multi-resonant therapeutic converter of claim 2 or claim 3, wherein at least one of the high-side transistor or the low-side transistor comprises a gate operable to receive a control signal.

5. The multi-resonant therapeutic converter of any one of claims 2 to 4, wherein the N resonant tanks comprise a first resonant tank and a second resonant tank, wherein the first resonant tank conducts current based on the high-side and low-side transistors being in a first state.

6. The multi-resonant therapeutic converter of claim 5, wherein the second resonant tank abstains from conducting current based on the high-side and low-side transistors being in the first state.

7. The multi-resonant therapeutic converter of claim 5 or claim 6, wherein, based on the high-side and low-side transistors being in a second state:
the second resonant tank conducts current; and
the first resonant tank abstains from conducting current.

8. The multi-resonant therapeutic converter of any one of claims 5 to 7, wherein the N resonant tanks further comprise a third resonant tank that conducts current based on the high-side and low-side transistors being in a third state, optionally wherein the first and second resonant tanks abstain from conducting current based on the high-side and low-side transistors being in the third state.

9. The multi-resonant therapeutic converter of any preceding claim, further comprising a clutching component coupled to the coil in parallel, optionally wherein the clutching component comprises a clutching inductor.

10. The multi-resonant therapeutic converter of claim 9, further comprising a clutching capacitor coupled in parallel to the coil and the clutching inductor.

11. The multi-resonant therapeutic converter of claim 9, further comprising a clutching resistor coupled in parallel to the coil, the clutching inductor, and the clutching capacitor.

12. The multi-resonant therapeutic converter of any one of claims 9 to 11, further comprising a solid-state relay (SSR) coupled in series with the clutching component.

13. A surgical system comprising:
a surgical instrument operable to perform a first operation and a second operation;
a multi-resonant therapeutic converter including a first resonant tank associated with the first operation and a second resonant tank associated with the second operation; and
a controller operable to selectively power the surgical instrument with the first and second resonant tanks.

14. The surgical system of claim 13, wherein the controller is operable to receive an input indicative of: (i) an applied voltage to the multi-resonant therapeutic converter; (ii) a voltage applied to the surgical instrument.

15. The surgical system of claim 13 or claim 14, wherein the multi-resonant therapeutic converter further includes a third resonant tank, and wherein the controller is operable to:
detect a change in frequency of the voltage applied to surgical instrument while performing the first operation; and
power the surgical instrument with the third resonant tank based on the change.
